# EUROPEAN PATENT APPLICATION

(11) **EP 2 966 881 A1**
(43) Date of publication of application: **13.01.2016**
(21) Application number: 14176716.0
(22) Date of filing: 11.07.2014
(51) Int. Cl.: H04R 25/00, A61B 5/12

(54) **Hearing device with ear monitoring function**

(71) Applicant: Oticon A/S, 2765 Smørum (DK)
(72) Inventor: Nielsen, Claus, DK-2765 Smørum (DK); Thorèn, Elisabeth Sundewall, DK-2765 Smørum (DK); Jensen, Lisbeth Dons, DK-2765 Smørum (DK); Hietkamp, Renskje K., DK-2765 Smørum (DK)
(74) Representative: Nielsen, Hans Jørgen Vind

(57) **Abstract**

According to an aspect, a hearing device adapted to be worn at an ear of a user for in-situ monitoring of a middle ear of the user is disclosed. The hearing device includes a receiver adapted to direct an incident electrical acoustic signal of a predetermined level and a predetermined frequency toward a tympanic membrane of the user; a measurement microphone adapted to covert a reflected sound signal that is reflected from the tympanic membrane in response to the incident electrical acoustic signal to a reflected electrical acoustic signal; a comparator adapted to calculate a level difference between the predetermined level and a reflected level of the reflected electrical acoustic signal; and a notification generator adapted to generate an indicator signal representative of status of the middle ear of the user if the level difference is equal or more than a predetermined threshold level.

## Description

### FIELD

The disclosure relates to a device having an ear monitoring functionality. In particular, the disclosure relates to a hearing device that is adapted to monitor status of a middle ear of a user of the hearing device.

### BACKGROUND

The middle ear is an air-filled cavity located behind the eardrum (tympanic membrane). The tympanic membrane acts as a natural boundary between the middle ear and the ear canal. Neutral air pressure in the middle ear is maintained by a narrow passage auditory tube (Eustachian tube), which connects the middle ear to the back of nose/ throat (nasopharynx). The Eustachian tube keeps the middle ear space at normal atmospheric pressure. The Eustachian tube is usually closed, but may open briefly to let some air through into the middle cavity for example, while swallowing or nose blowing. It also tends to open when there are quick changes in external atmospheric pressure, such as when travelling in a lift, driving up or down a steep hill, and in an aircraft during take-off or landing. This allows fresh air to enter the middle ear. The fresh air allows for equalizing the pressure between the middle ear and the outside environment. Thus, Eustachian tubes play an important role in pressure equalization.

A common cause of middle ear disorder is Eustachian tube dysfunction. If the Eustachian tube becomes blocked by swelling or congestion in the nose and throat, or by swelling of the mucous membrane in the middle ear, or by swelling of the mucous membrane of the Eustachian tube itself, then the air pressure in the middle ear cannot equalize properly. This causes the mucosal lining of the middle ear cavity to absorb the trapped air, thus creating a vacuum. This pressure differential causes the tympanic membrane to retract inwards, which tends to stiffen the middle ear system, leading to a slight or mild hearing loss. This negative middle ear pressure often is a forerunner to Otitis Media (OM), whereby the middle ear cavity fills with fluid, leading to greater conductive hearing loss. Children tend to be far more susceptible to Eustachian tube dysfunction than adults.

The Eustachian tube also serves to drain mucus or fluid from the middle ear. If the Eustachian tube blockage persists, chronic changes in the tissue of the middle ear begin to occur. Firstly, the ability of the Eustachian tube to act as an efficient drain diminishes and the mucous secretions become thicker, and therefore less likely to drain. Then the membranes themselves begin to thicken and become inflamed. The defense mechanisms of the Eustachian tube and middle ear become compromised and bacteria normally present in the nose may enter the middle ear and cause a painful condition called acute OM. This condition is usually treated by antibiotics and may even require treatment by inserting small tubes (called ventilation tubes) into the tympanic membrane, facilitating drainage of any accumulated fluid in the middle ear. Unfortunately, the insertion of ventilation tubes in this manner requires invasive surgical intervention. In addition, the use of ventilation tubes also increases the risk of infection from the environment.

The negative pressure in the middle ear or alternating periods of negative, normal and positive pressure may deform the eardrum. In the long term, the eardrum may become severely distorted, thinned, or may even be perforated. These changes may even cause permanent hearing loss. Sudden changes to the middle ear status may result in up to 30 dB extra hearing loss across the frequency range. Younger children such as between 1 and 6 years easily contract virus and infections, which may cause changes to the middle ear status. Parents, teachers and other people around the child typically fail to notice the exact condition of child's middle ear. Such an extra 10-30 dB hearing loss is problematic for children and may invalidate learning as well.

Complications of undiagnosed OM may include hearing loss; left untreated, OM may lead to behavioural, educational, speech and language development delays. Inflammation and infection in time may cause erosion of the ossicles and the walls of the middle and inner ear. The patient may experience hearing loss, imbalance, or weakness of facial movement on the affected side.

Hearing care practitioners use a variety of tests to determine status of the middle ear. However, such procedures are undertaken only occasionally because it requires the child to be taken to a hearing care professional for tests and the analysis depends upon practitioner's experience and co-operation of the child during the tests. Therefore, a more effective middle ear status monitoring is required.

### SUMMARY OF THE DISCLOSURE

According to an aspect, a hearing device adapted to be worn at an ear of a user for in-situ monitoring of a middle ear of the user is disclosed. The hearing device includes a receiver adapted to direct an incident electrical acoustic signal of a predetermined level and a predetermined frequency toward a tympanic membrane of the user; a measurement microphone adapted to covert a reflected sound signal that is reflected from the tympanic membrane in response to the incident electrical acoustic signal to a reflected electrical acoustic signal; a comparator adapted to calculate a level difference between the predetermined level and a reflected level of the reflected electrical acoustic signal; and a notification generator adapted to generate an indicator signal representative of status of the middle ear of the user if the level difference is equal or more than a predetermined threshold level.

According to another aspect, a method for in-situ monitoring of an ear of a user wearing a hearing device is disclosed. The method includes measuring a level difference between a predetermined level of an incident electrical acoustic signal and a reflected level of a reflected acoustic signal, the incident electrical acoustic signal of the predetermined level and a predetermined frequency being impinged on a tympanic membrane of the user and the reflected electrical acoustic signal being reflected from the tympanic membrane in response to the incident electrical acoustic signal; and activating an indicator means using an indicator signal representative of status of a middle ear of the user if the level difference is equal or more than a predetermined threshold level that is stored in a memory of the hearing device.

### BRIEF DESCRIPTION OF ACCOMPANYING FIGURES

The embodiments of the disclosure, together with its advantages, may be best understood from the following detailed description taken in conjunction with the accompanying figures in which:
Fig. 1 illustrates a hearing device for in-situ monitoring of the ear of a user wearing the hearing device according to an aspect of the disclosure;
Fig. 2 illustrates the hearing device of Fig. 1 for in-situ monitoring of the ear of a user wearing the hearing device according to an aspect of the disclosure;
Fig. 3 illustrates a measurement microphone housed in an ear mould according to an aspect of the disclosure;
Fig. 4 illustrates an incident electrical acoustic signal according to an aspect of the disclosure;
Fig. 5 illustrates a plurality of incident electrical acoustic signals according to an aspect of the disclosure;
Fig. 6 illustrates a method for an in-situ monitoring of an ear of a user wearing a hearing device according to an aspect of the disclosure; and
Fig. 7 illustrates a method for an in-situ monitoring of the ear of a user wearing the hearing device according to an aspect of the disclosure.

### DETAILED DESCRIPTION

A hearing device adapted to be worn at an ear of a user for in-situ monitoring of a middle ear of the user is defined in claim 1 along with related independent method claim. Other aspects are defined in the dependent sub-claims and also explained in the following description and illustrated in the accompanying drawings.

In different embodiments, the hearing device refers to an apparatus such as e.g. a hearing aid, headset, headphone, ear phone or other devices that deliver sound to the ear while the apparatus is being worn by the user. Such other devices may include apparatus that is only used for middle ear status monitoring purposes and not for improving hearing capabilities. However, some of these devices like the hearing aids are typically used to augment and/ or improve hearing capabilities of a user. This is typically performed by the apparatus having an input transducer for receiving sound signals from the user's surroundings and generating corresponding electrical input audio signal or directly receiving an electronic audio signal, a signal processing circuit for processing the input audio signal or the electronic audio signal and possibly modifying the electrical audio signals and an output means like receiver for providing the possibly modified electrical/ electronic audio signals as modified sound signals to at least one of the user's ears. Some hearing devices may comprise multiple input transducers, e.g. for providing direction-dependent audio signal processing.

The hearing device may comprise a single unit or several units communicating electronically with each other such as in a binaural hearing aid system. Each of the one or more units of a hearing device may be configured to be worn in any known way, e.g. behind the ear, entirely or partly arranged in the pinna and/or in the ear canal, as an entirely or partly implanted unit, etc.

A "hearing system" refers to a system comprising one or two hearing devices, and a "binaural hearing system" refers to a system comprising one or two hearing devices and being adapted to cooperatively provide audible signals to both of the user's ears. In a hearing system or a binaural hearing system, both of the hearing devices may comprise individual output means in order to provide audible signals. Hearing systems or binaural hearing systems may further comprise "auxiliary devices", which communicate with the hearing devices and affect and/or benefit from the function of the hearing devices. Auxiliary devices may be e.g. remote controls, remote microphones, audio gateway devices, mobile phones, public-address systems, car audio systems or music players. Hearing devices, hearing systems or binaural hearing systems may e.g. be used for compensating for a hearing-impaired person's loss of hearing capability, augmenting or protecting a normal-hearing person's hearing capability and/or conveying electronic audio signals to a person.

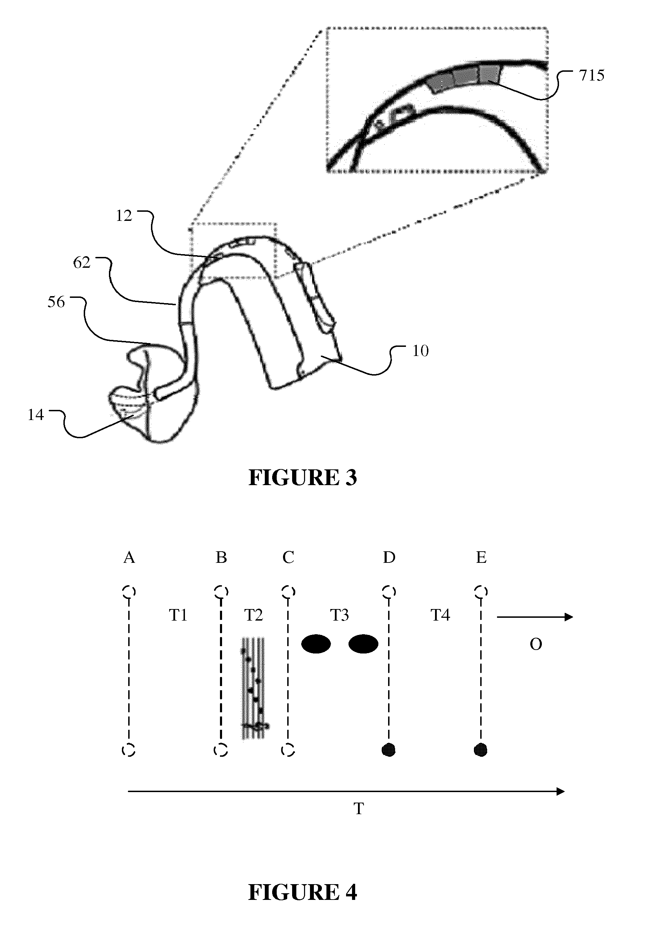

In different embodiments, the term "worn" refers to the hearing device such as a hearing aid being positioned at the user's ear such that sound may be delivered to user's ear. For example, when a Behind-the-Ear (BTE) hearing aid type is worn, a main body of the BTE sits behind the pinna of the user and the sound is transmitted from a receiver housed in the main body through a sound tube (62, Fig. 3) to the ear canal of the user. In other examples, when In-the-Ear (ITE) or Completely-in-Canal (CIC) type of hearing aids are worn, the receiver is positioned within the ear canal and the sound is transmitted directly, without using the sound tube, from the receiver to the ear canal of the user. For the hearing system, one hearing device may be arranged at a left ear and one hearing device can be arranged at a right ear of the user with an insertion part having the output acoustic transducer (receiver) of the hearing device being arranged in an ear canal of the user.

In different embodiments, the term "in-situ" refers to using the hearing device for monitoring status of the ear when the hearing device is in use, i.e. worn by the user. In one aspect, the in-situ monitoring includes monitoring of the middle ear with the hearing device operating in a middle ear status monitoring mode (described later), the hearing device being the same hearing device that the user wears for sound delivery when the hearing device operates in a hearing aid mode (described later). Furthermore, the positioning of the hearing device at the ear when used for in-situ monitoring corresponds to the position of the hearing device when the hearing device is used sound delivery in the hearing aid mode.

Although it is preferable to use the same hearing device for monitoring as well as for hearing purposes. However, in another aspect, the hearing device is the monitoring apparatus only without having functionalities of a conventional hearing aid, the monitoring apparatus is adapted to be worn at the ear of the user at the same position where the hearing device is usually positioned for sound delivery.

The disclosure generally utilizes the principle - when the tympanic membrane is hit by an incident sound, part of the sound is absorbed and sent via middle ear to the inner ear while the other part of the sound is reflected. The stiffer the tympanic membrane, the more sound is reflected and the less sound reaches the inner ear. A comparison between the incident sound and the reflected sound allows for determining the status of the tympanic membrane and thus, of the middle ear.

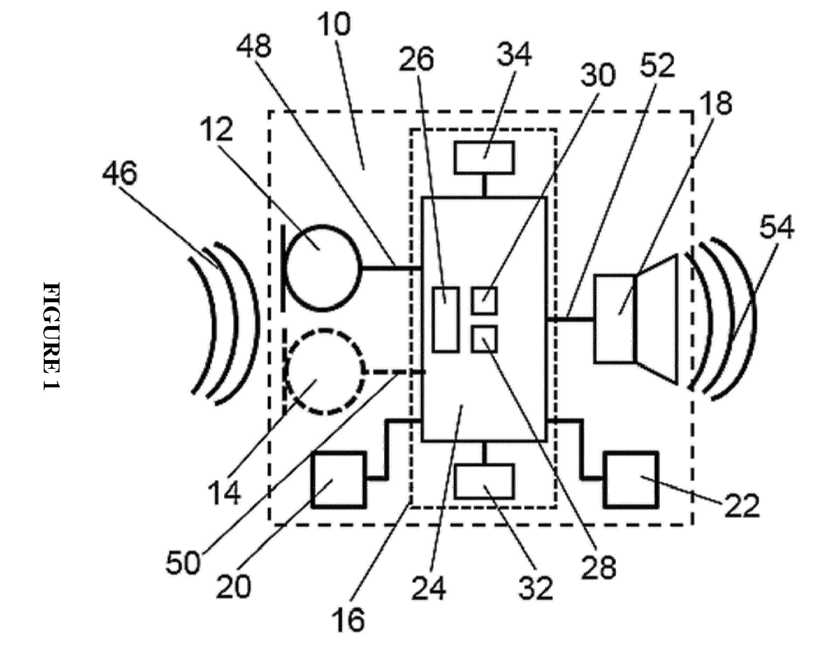

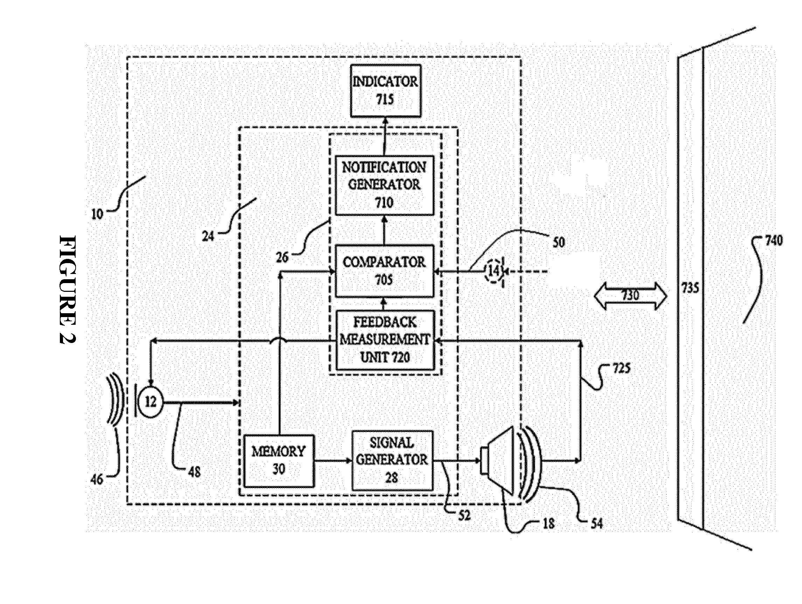

Referring now to Fig. 1 that illustrate the hearing device 10 used for in-situ monitoring of the ear of a user wearing the hearing device according to an aspect of the disclosure. Reference is also made to Fig. 2 that illustrates in further detail the hearing device 10 of Fig. 1.

The hearing device 10 is adapted to be worn at the ear of the user for in-situ monitoring of the middle ear (represented by side 740) of the user. The hearing device includes an acoustic output transducer (receiver) 18 adapted to direct an incident electrical acoustic signal 52 of a predetermined level and a predetermined frequency toward a tympanic membrane 735 of the user. A measurement microphone 14 is adapted to covert a reflected sound signal 730, reflected from the tympanic membrane 735 in response to the incident electrical acoustic signal 52, to a reflected electrical acoustic signal 50. The device further includes a comparator 705 adapted to calculate a level difference between the predetermined level of the incident electrical acoustic signal 52 and a reflected level of the reflected electrical acoustic signal 50; and a notification generator 710 adapted to generate an indicator signal representative of status of the middle ear 740 of the user if the level difference is equal or more than a predetermined threshold level.

In various embodiments, the predetermined frequency is selected from a range between 1 KHz to 4 KHz. For example, the predetermined frequency may include a frequency such as 250 Hz, or 500 Hz, or 1000 Hz, or 2000 Hz, or 4000 Hz. Other frequencies within the range is also possible.

The hearing device 10 may additionally include a microphone 12 that is adapted to capture speech and/ or ambient (environment) sound signals. In the disclosed embodiment, the measurement microphone 14 is different from the microphone 12.

The hearing device may further include an electric circuitry 16, a user interface 20 and a battery 22. The electric circuitry 16 comprises a control unit 24, a processing unit 26, a signal generator 28, a memory 30, a receiver unit 32, and a transmitter unit 34. The processing unit 26 is adapted to include the comparator 705, and notification generator 710. A feedback measurement unit 720 may also be included. In this embodiment, the processing unit 26, the signal generator 28 and the memory 30 are part of the control unit 24.

The hearing device 10 is configured to operate in various modes of operation, which the control unit 24 executes while using various components of the hearing device 10. For example, in the hearing aid mode, the hearing device 10 is used as a hearing aid for hearing improvement by sound amplification and filtering. Whereas, in a middle ear monitoring mode, the same hearing device 10 is adapted to determine the status of the middle ear. The processing unit 26 is therefore adapted to execute appropriate algorithms for each of these modes. This may include at least one or more of applying outputs on electrical signals processed by the processing unit 26, and to perform calculations, e.g., for filtering, for amplification, for signal processing, feedback measurement, signal comparison, notification generation or for other functions. Executing the modes of operation includes the interaction of various components of the hearing device 10.

Additionally, an activation unit (not shown) may be used to instruct the hearing device 10 to select a mode of operation for example the hearing aid mode. Similarly, the activation unit is adapted to instruct the hearing device to operate in the middle ear status monitoring mode, i.e. to perform an in-situ measurement of the level difference at a predetermined event. In some embodiments, the activation unit may form part of the processing unit 26, the activation unit being adapted to communicate with other components like a receiver unit 32, and/ or user interface 20 to determine the occurrence of the predetermined events. Once the activation unit receives notification of the activation signal, the activation unit instructs the processor to execute appropriate algorithms to operate the hearing device in the middle ear status monitoring mode. The predetermined events typically include when the hearing device is switched on and/ or when the activation unit is notified reception of an activation signal at the receiver unit 32 from an external device such as an smartphone running an mobile app that allows communication with the hearing device, and/ or when the activation unit is notified reception of an activation signal at the user interface 20 based on the user interaction directly with the hearing device. The user interface may include both physical buttons or a touch sensitive controls like on a smartphone screen or any other functionally comparable means.

The hearing device 10 operating in the hearing aid mode receives environment sound 46 with using microphone 12. The microphone 12 generates electrical environment sound signals 48, which are provided to the control unit 24. The processing unit 26 of the control unit 24 processes the electrical sound signals 48, e.g. by spectral filtering, frequency dependent amplifying, filtering, or other typical processing of electrical sound signals in a hearing aid generating an output sound signal 52. The processing of the electrical sound signals 48 by the processing unit 26 depends on various parameters, e.g., sound environment, sound source location, signal-to-noise ratio of incoming sound, setting of the hearing device, type of output transducer, battery level, and/or other user specific parameters and/or environment specific parameters. The output sound signal 52 is provided to the speaker 18, which generates an output sound 54 corresponding to the output sound signal 52, stimulating hearing of the user. Even though only one microphone 12 for this purpose is shown, it is conceivable to have multiple microphones for improved performance.

The hearing device 10 operating in the middle ear monitoring mode directs an incident electrical acoustic signal 52 of a predetermined level and a predetermined frequency as an incident sound 54 toward a tympanic membrane 735 of the user using a receiver 18. The measurement microphone 14 is adapted to covert a reflected sound 730, reflected from the tympanic membrane 735 in response to the incident electrical acoustic signal 52, to a reflected electrical acoustic signal 50. A comparator 705 is adapted to calculate a level difference between the predetermined level and a reflected level of the reflected electrical acoustic signal; and a notification generator 710 adapted to generate an indicator signal representative of status of the middle ear of the user if the level difference is equal or more than a predetermined threshold level. It is to be noted that in this aspect, representative numeral 52 is used for showing incident electrical acoustic signal and should not be misinterpreted to be representing output sound signal of the hearing aid mode. Similarly, in this aspect, numeral 54 represents the incident sound corresponding to the incident electrical acoustic signal and should not be misinterpreted to be representing output sound of the hearing aid mode.

The comparator 705 may include a number of sub-components determining the level difference. In one aspect, an absolute value determination unit determines magnitude or magnitude squared of the respective incident electrical acoustic signal 52 and reflected electrical acoustic signal 50. The filters may be used to determine a level based on a short term basis, such as a level based on a short time interval, such as for example the last 5 ms to 40 ms or such as the last 10 ms. The level may then be converted to a domain such as a logarithmic domain or any other domain. Then, a summation unit may be used to determine a level difference. The level difference is calculated for each time period and the predetermined frequency of the incident electrical acoustic signal 52. The skilled person would find it reasonable to implement the comparator for determining level difference using a number of conventionally known techniques. In another aspect, the predetermined level of the incident electrical acoustic signal at the predetermined frequency is already available in the hearing device such as stored in the memory 30 and therefore, the level determination for comparison between the predetermined level and the reflected level only requires determining the reflected level of the reflected electrical acoustic signal.

In various aspects, the predetermined threshold level is calculated in different ways. The threshold level difference includes at least one of a fitting level difference, an adjusted level difference, and a default level difference. Some of these implementations relate to a hearing device fitting session. The fitting session typically includes a hearing care professional (HCP) using a fitting software to adjust hearing aid device so that the device output in the hearing device user's ear matches a prescribed target, which is a function of a response curve of the user. During the fitting session, in these implementations, the predetermined threshold may also be determined as described below.

In a first aspect, a fitting level difference is determined between the predetermined level of the incident electrical acoustic signal 52 and a fitting level of a fitting electrical acoustic signal during a hearing aid fitting session of the user, the incident electrical acoustic signal 52 of the predetermined level and the predetermined frequency being impinged on the tympanic membrane 735 of the user and the fitting electric acoustic signal being reflected from the tympanic membrane 735 in response to the incident electrical acoustic signal 52. If during the fitting session the HCP determines that the fitting level difference shows normal working of the middle ear, then the fitting level difference becomes the predetermined threshold level for in-situ monitoring of the middle ear where the same incident electrical acoustic signal 52 with same predetermined level and predetermined frequency as used during the fitting session is used as the incident electrical acoustic signal 52 for in-situ monitoring.

In another aspect, an adjusted level difference comprising a fitting level difference between the predetermined level of the incident electrical acoustic signal 52 and a fitting level of a fitting electrical acoustic signal during a hearing aid fitting session of the user wherein the fitting level difference being adjusted in accordance with a defined rule, the incident electrical acoustic signal 52 of the predetermined level and the predetermined frequency being impinged on the tympanic membrane 735 of the user, the fitting electric acoustic signal being reflected from the tympanic membrane 735 in response to the incident acoustic signal 52. This implementation is same as the one described above, except that in this implementation, the fitting level difference is adjusted in accordance with a defined rule. The rule may include increasing the fitting level difference to a value higher than the determined fitting level difference. However, even with adjustment, the higher value is lower than or equal to a level that the HCP would classify as representative of middle ear problem/ disorder. Alternatively, for a specific user, the HCP may decide to alter the rule so that the fitting level difference is adjusted to a value lower than the determined fitting level difference. This might be useful when during the fitting session, the user already demonstrates a middle ear disorder. Therefore, the adjusted level difference becomes the predetermined threshold level for in-situ monitoring of the middle ear where the same incident electrical acoustic signal 52 with same predetermined level and predetermined frequency as used during the fitting session is used as the incident electrical acoustic signal 52 for in-situ monitoring.

In yet another aspect, a default level difference comprising a level difference stored in the memory of the hearing device is used. The level difference is selected from a library comprising an average level difference for a user group. The default level difference may be stored during different stages for example, during manufacturing of hearing device or during programming of the hearing device at the fitting session. The average level difference is average of the level difference between the incident electrical acoustic signal 52 of the predetermined level and predetermined frequency and reflected level of the reflected electrical acoustic signal in response to the incident electrical acoustic signal, for each individual in the user group. The user group represents a sample that is big enough to provide high confidence in the average level difference and that includes user profile share substantial subjective and/ or objective characteristics with the user. The characteristics are defined in terms of both objective parameters like age, gender, etc. and/ or subjective parameters like city v. sub-urban dwelling, sports involved in, etc.. The average level difference may also include weighted average level difference where level difference from individuals of the user group sharing a certain type characteristics with the user is given relatively higher weight in determining the average level difference.

The memory 30 is adapted to store the predetermined threshold level for the incident acoustic electrical signal. The memory 30 may also be adapted to store an executable file corresponding to the incident acoustic electrical signal of the predetermined level and the predetermined frequency. The signal generator 28 is adapted to render the executable file and produce the incident acoustic electrical signal that is delivered to the tympanic membrane 735 as the incident sound 54 using the receiver 18.

In another aspect, the memory 30 is adapted to store at least one predetermined threshold level for at least one incident electrical acoustic signal. Each incident electrical acoustic signal 52 of the at least one electrical acoustic signal is of a specific level and a specific frequency within a frequency range. The incident electrical acoustic signal of the predetermined level and predetermined frequency being selected from the at least one electrical acoustic signal. It is understandable that for the incident acoustic signals may include different incident electrical acoustic signals that may have same frequency-different level or different frequency-different level, or different frequency-same level.

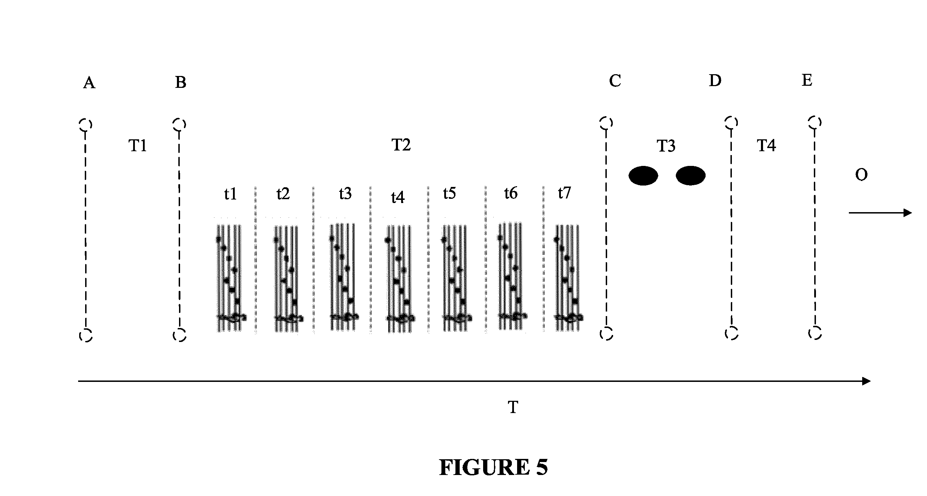

In an embodiment, the signal generator 28 may be adapted to generate a plurality of incident electrical acoustic signals with each incident electrical acoustic signal of the plurality of incident electrical acoustic signals being impinged for a predefined time-period (see Fig. 5), the each incident electrical acoustic signal 52 of the plurality of the incident electrical acoustic signals being of a specific level and of a specific frequency within a frequency range; and the memory 30 is adapted to store a plurality of threshold levels corresponding to each specific level and each specific distinct frequency and/ or a plurality of threshold feedback values corresponding to each specific level and each specific distinct frequency. The specific frequency may include a frequency that is selected from a range between 1 KHz to 4 KHz. It may be envisaged that each incident electrical acoustic signal of the plurality of incident electrical acoustic signals may have same frequency-different level or different frequency-different level, or different frequency-same level.

The comparator may be adapted to calculate a plurality of level differences between the specific level of each of the plurality of the incident electrical acoustic signal and a plurality of reflected levels corresponding to a plurality of reflected electrical acoustic signals; and a notification generator adapted to generate an indicator signal representative of status of a middle ear of the user based on the threshold level difference corresponding to each of the plurality of incident electrical acoustic signal or a rule based average threshold level difference corresponding to the plurality of incident electrical acoustic signals. For example, in the latter aspect, it may be suitable that level difference relating to particular frequency may be preferentially "weighted" compared to other frequencies to provide improved statistical analysis. In this manner, important frequency values or ranges can be afforded more weight, so that differences between measured reflected level and the predetermined threshold level is emphasized compared to other differences that may be less pertinent for a particular situation.

The notification generator 710 is adapted to transmit the indicator signal to an indicator 715 for producing a perceivable effect. The comparator 705 generates the indicator signal when the level difference is equal or more than the predetermined threshold level. The indicator signal is provided to the indicator that is adapted to produce the perceivable effect. Although the indicator 715 is shown as part of the hearing device but in other aspects, the indicator 715 may be remotely located device such as a smartphone. In these aspects, the transmitter unit 34 of the hearing device is capable of transmitting the indicator signal to the remotely located device where the perceivable effect is produced. The perceivable effect is selected from a group consisting of a visual signal like LED lighting up or blinking at the hearing aid device/ remote device like a smartphone, a vibration of the hearing aid device/ remote device like the smartphone, an audio signal using the receiver of the hearing aid device/ speaker of a remote device like a smartphone, text signal at the remote device like smartphone. The indicator 715 may be adapted to produce the perceivable effect that is a function of the magnitude of level difference above the predetermined threshold level. For example, if the level difference is marginally high, the indicator produces an ORANGE light whereas for a level difference that is much higher, the indicator produces a RED light. It may also be envisaged that the notification generator 710 is also adapted to generate a positive indicator signal representative of status of the middle ear 740 of the user if the level difference is below the predetermined threshold level. For example, if the perceivable effect when the level difference is equal or more than the predetermined threshold level then the indicator 715 continuously blinks RED LED light, then for the level difference below the predetermined threshold level the indicator 715 is adapted to light up a GREEN LED light.

Fig. 4 illustrates an incident electrical acoustic signal according to an aspect of the disclosure. At time point B, the incident sound 54 corresponding to the incident electrical acoustic signal 52 is played for a predefined timeperiod T2 until time point C. Following time period T2, the indicator 715 will produce a perceivable effect for the time duration T3 until time point D if the determined level difference is equal or above the predetermined threshold level. It may also be possible that a different perceivable effect is produced during time duration T3 if the middle ear status is normal. This is helpful in ascertaining that the monitoring steps did take place.

Fig. 5 illustrates a plurality of incident electrical acoustic signals according to an aspect of the disclosure. In this aspect, at time point B, a plurality of incident sound corresponding to a plurality of incident electrical acoustic signals are sequentially played during a predefined timeperiod T2 until time point C. Following time period T2, the indicator 715 will produce a perceivable effect for the time duration T3 until time point D if the level difference meets the activation criteria (described later). Like the earlier aspect, it may be useful to produce a different perceivable effect during time duration T3 if the middle ear status is normal.

In aspects described above, time period T1 may be boot up time, i.e. the hearing device is switching on. It may even be a time during which the hearing device is being used for example for a hearing aid the use includes using the hearing device in the hearing aid mode and at time point B, the activation unit receives notification of an activation signal for performing the in-situ monitoring. Similarly, T4 may represents reboot time after the performance of the in-situ monitoring, i.e. for a hearing aid operating the hearing aid in the middle ear status monitoring mode, followed by usual use of the hearing device from time point E onwards. Additionally or alternatively, it may also represent using the hearing device without rebooting and using the hearing device in usual usage mode from time point D onwards.

In one aspect, the processor 26 is adapted to adjust at least one parameter, such as gain, of the hearing device based on the measured level difference if the level difference is equal or more than a predetermined threshold level. Because higher level difference indicates lesser sound for same incident electrical acoustic signal being transmitted to the middle ear and the inner ear; therefore in order to produce the same perception of sound to the user the processor 26 may provide higher gain to sound captured by the microphone 12. In order to achieve this, the control unit 24 is adapted to use the level difference from the middle ear status monitoring mode, as an input to parameter adjustment algorithm that is used in the hearing aid mode. In the hearing aid mode, the processor executes the parameter adjustment mode accordingly and adjusts the parameter such as gain to counter the loss of perception of sound because of the changed level difference. However, the adjustment of the parameter of the hearing device based on the determined level difference is typically within a permissible range, the permissible range being such that the higher value of the range is not uncomfortable and/ or dangerously high for the user.

In an aspect, the hearing device 10 is adapted to utilize feedback measurement to determine status of middle ear. Feedback occurs in the hearing device 10 when the output sound 54 from the hearing device propagates beside an ear mould (fig. 3, 56) or through a vent and consequently enter the hearing aid microphone 12 as an acoustic input, as part of sound 46. The size and shape of the earmold may vary depending upon anatomy of user's ear canal in order to seal the user's ear canal. With higher reflection from the tympanic membrane, the feedback value may increase suddenly. Therefore, a feedback measurement unit 720 may be provided, the unit is adapted to measure the feedback 725 at the microphone 12 for the incident electrical acoustic signal of the predetermined level and predetermined frequency and/ or the electrical sound signal 48. The comparator is adapted to detect whether a change in the feedback value is equal or more than a threshold feedback value corresponding to the incident electrical acoustic signal and/ or the electrical sound signal 48; and the notification generator adapted to generate the indicator signal representative of status of the middle ear of the user when the feedback value change is equal or more than the threshold feedback value. The memory is adapted to store the threshold feedback value, the feedback measurement unit 720 measures the feedback continually or continuously and the comparator 705 compares a feedback value with a preceding feedback value and calculates the change in the feedback value. The comparator 705 is also adapted to access the threshold feedback value and determine whether the calculated change in the feedback value is equal or more than the threshold feedback value. If so, then the comparator 710 instructs the notification generator 710 to generate an indication signal to produce a perceivable effect at the indicator 715. The skilled person would understand and unambiguously derive that the role of notification signal generator, comparator, indicator signal, and indicator are similar to the earlier embodiments and some of the features from earlier embodiment that are not explicitly recited here are still applicable. For example changing the perceivable effect based on the level of change, etc. The difference in this embodiment and earlier embodiments is that here the comparison and status monitoring is based on change in feedback value instead of level difference between the incident and reflected electrical acoustic signals.

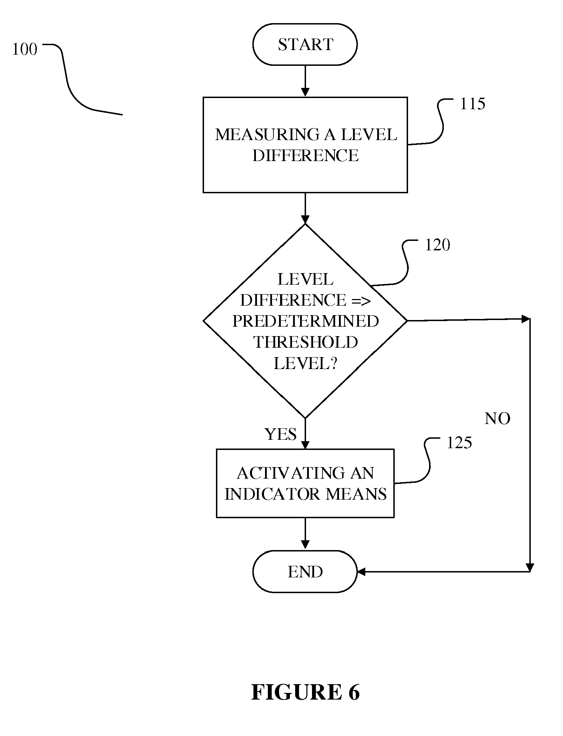

According to an aspect, Fig. 6 illustrates a method 100 for in-situ monitoring of an ear of a user wearing a hearing device. The method includes, at 115, measuring a level difference between a predetermined level of the incident electrical acoustic signal and a reflected level of a reflected acoustic signal. The incident electrical acoustic signal is of the predetermined level and a predetermined frequency and is impinged on a tympanic membrane of the user and the reflected electrical acoustic signal is reflected from the tympanic membrane in response to the incident electrical acoustic signal. At 125, an indicator is activated using an indicator signal representative of status of a middle ear of the user if the level difference is equal or more than a predetermined threshold level that is stored in a memory of the hearing device, as illustrated at 120.

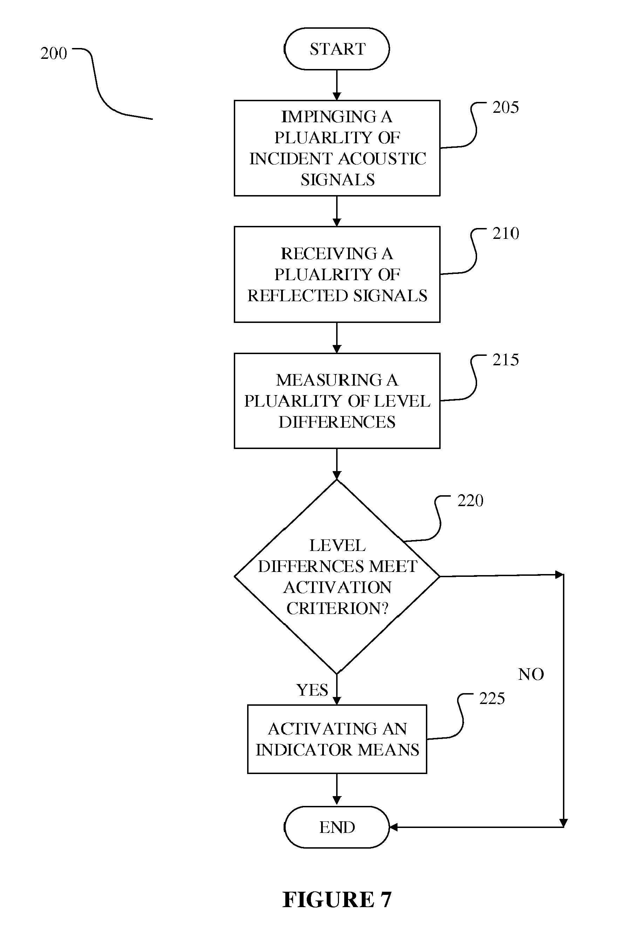

According to another aspect, as illustrated in Fig. 7 additional steps may also be included. At 205, a plurality of incident electrical acoustic signals are sequentially directed towards the tympanic membrane of the user. Each signal of the plurality of the incident electrical acoustic signals is of a specific level and of a specific frequency. In response to the each incident electrical acoustic signal, a plurality of reflected signals are received at a measuring microphone at 210. At 215, the reflected level of each of the reflected signals is compared with the specific level of each of corresponding incident electrical acoustic signal to obtain a plurality of level differences. At 220, a determination is made whether the plurality of level differences meet the criteria for activating the indicator. And, at 225, the indicator is activated if the level difference meets the activation criteria.

In one aspect, the activation criteria may include whether the determined level difference is equal or more than the threshold level difference for each of the incident electrical acoustic signal. In another aspect, the criteria may include whether the determined level difference is equal or more than the threshold level difference for more than a specified percent such as 40% or 50% or 60% or 70% or 80% of the incident electrical acoustic signals. In yet another aspect, the criteria may include whether the determined level difference is equal or more than the a weighted level difference for the plurality of incident electrical acoustic signals. For example, it may be suitable that level difference relating to particular frequency may be preferentially "weighted" compared to other frequencies to provide improved statistical analysis. In this manner, important frequency values or ranges can be afforded more weight, so that differences between measured reflected level and the predetermined threshold level is emphasized compared to other differences that may be less pertinent for a particular situation.

It is understood that the each incident electrical acoustic signal of the plurality of the incident electrical acoustic signals is impinged for a predefined time period. It may be envisaged that different incident electrical acoustic signal of the plurality of incident electrical acoustic signals may have same frequency-different level or different frequency-different level, or different frequency-same level.

In an aspect, the incident electrical acoustic signal is impinged using a receiver of the hearing aid device for a predefined time duration and the reflected electrical acoustic signal is received at a measurement microphone of the hearing aid device, the measurement microphone being different from a microphone of the hearing aid device adapted to capture speech and/ or ambient sound signals. In an aspect, the measurement microphone 14 is included in a mould (Fig. 3, 56) that is inserted inside the ear canal of the user. It is apparent that the size and shape of an earmold may vary, depending on several factors in accordance with anatomy of user's ear canal in order to seal the user's ear canal. There are a number of materials that are known and may be used. For example, the materials used for soft type moulds are silicone and vinyl, and for hard types are lucite and acrylic. Ear mould may also be made of a blend of several of these materials. In another aspect, the incident electrical acoustic signal is impinged using the receiver of the hearing device for a predefined time duration and the reflected acoustic signal is received at the microphone 12 of the hearing device, further comprising generating the indicator signal representative of status of the middle ear of the user when the feedback value change is equal or more than the threshold feedback value.

In yet another embodiment, an indicator rule may be defined that is used to activate the indicator for notifying the status of the middle ear. The rule may include a combination of the result produced by a) level difference based activation of the indictor, and b) change in feedback measurement based activation of the indicator. The combination may include a) activating the indicator when the level difference is equal or more than the predetermined threshold value and the feedback value change is equal or more than the threshold feedback value, or b) when either the level difference is equal or more than the predetermined threshold value or the feedback value change is equal or more than the threshold feedback value, or c) when the level difference is equal or more than the predetermined threshold value and the feedback value change is lower than the threshold feedback value but within a certain range of the threshold feedback value, or d) when the feedback value change is equal or more than the threshold feedback value and when the level difference is lower than the predetermined threshold value but within a certain range of the predetermined threshold value.

It should be appreciated that reference throughout this specification to "one embodiment" or "an embodiment" or features included as "may" means that a particular feature, structure or characteristic described in connection with the embodiment is included in at least one embodiment of the disclosure. Therefore, it is emphasized and should be appreciated that two or more references to "an embodiment" or "one embodiment" or "an alternative embodiment" or features included as "may" in various portions of this specification are not necessarily all referring to the same embodiment. Furthermore, the particular features, structures or characteristics may be combined as suitable in one or more embodiments of the disclosure.

Throughout the foregoing description, for the purposes of explanation, numerous specific details. were set forth in order to provide a thorough understanding of the disclosure. It will be apparent, however, to one skilled in the art that the disclosure may be practised without some of these specific details.

Accordingly, the scope should be judged in terms of the claims that follow.

## Claims

1. A hearing device adapted to be worn at an ear of a user for in-situ monitoring of a middle ear of the user, the hearing device comprising
a receiver adapted to direct an incident electrical acoustic signal of a predetermined level and a predetermined frequency toward a tympanic membrane of the user;
a measurement microphone adapted to covert a reflected sound signal, reflected from the tympanic membrane in response to the incident electrical acoustic signal, to a reflected electrical acoustic signal;
a comparator adapted to calculate a level difference between the predetermined level and a reflected level of the reflected electrical acoustic signal; and
a notification generator adapted to generate an indicator signal representative of status of the middle ear of the user if the level difference is equal or more than a predetermined threshold level.

2. The hearing device according to claim 1, further comprising
a memory adapted to store at least one predetermined threshold level for at least one incident electrical acoustic signal with each incident electrical acoustic signal of the at least one electrical acoustic signal of a specific level and a specific frequency within a frequency range, the incident electrical acoustic signal of the predetermined level and predetermined frequency being selected from the at least one electrical acoustic signal; and/ or
a signal generator adapted to generate the incident electrical acoustic signal of the predetermined level and predetermined frequency, the incident electrical acoustic signal having a predetermined time duration.

3. The hearing device according to any of the preceding claims, wherein the measurement microphone is housed in an ear mold of the hearing device, the measurement microphone being different from a microphone of the hearing device that is adapted to capture speech and/ or ambient sound signals.

4. The hearing device according to any of the preceding claims, wherein the predetermined threshold level comprises
a fitting level difference as determined between the predetermined level of the incident electrical acoustic signal and a fitting level of a fitting electrical acoustic signal during a hearing aid fitting session of the user, the incident electrical acoustic signal of the predetermined level and the predetermined frequency being impinged on the tympanic membrane of the user and the fitting electric acoustic signal being reflected from the tympanic membrane in response to the incident acoustic signal; or
an adjusted level difference comprising a fitting level difference between the predetermined level of the incident electrical acoustic signal and a fitting level of a fitting electrical acoustic signal during a hearing aid fitting session of the user wherein the fitting level difference being adjusted in accordance with a defined rule, the incident electrical acoustic signal of the predetermined level and the predetermined frequency being impinged on the tympanic membrane of the user, the fitting electric acoustic signal being reflected from the tympanic membrane in response to the incident acoustic signal; or
a default level difference comprising a level difference stored in the memory of the hearing device, the level difference is selected from a library comprising an average level difference for a user group.

5. The hearing device according to any of the preceding claims, further comprising an activation unit for instructing the hearing device to perform an in-situ measurement of the level difference at a predefined event.

6. The hearing device according to any of the preceding claims, wherein the predefined event is selected from a group consisting of when the hearing device is switched on, or when the activation unit receives notification of an activation signal from an external device, or when the activation unit receives notification of an activation signal based on a user interaction directly with the hearing device or a combination thereof.

7. The hearing device according to any of the preceding claims, further comprising
a feedback measurement unit adapted to measure the feedback at the microphone for the incident electrical acoustic signal of the predetermined level and predetermined frequency;
the comparator being adapted to detect whether a change in the feedback value is equal or more than a threshold feedback value corresponding to the incident electrical acoustic signal; and
the notification generator adapted to generate the indicator signal representative of status of the middle ear of the user when the feedback value change is equal or more than the threshold feedback value.

8. The hearing device according to any of the preceding claims, wherein
the signal generator is adapted to generate a plurality of incident electrical acoustic signals with each incident electrical acoustic signal of the plurality of incident electrical acoustic signals being impinged for a predefined time-period, the each incident electrical acoustic signal of the plurality of the incident electrical acoustic signals being of a specific level and of a specific frequency within a frequency range, the specific level being fixed or changeable for each predetermined frequency; and
the memory is adapted to store a plurality of threshold levels corresponding to each specific level and each specific frequency and/ or a plurality of threshold feedback values corresponding to each specific level and each specific frequency.

9. The hearing device according to any of the preceding claims, wherein
the comparator is adapted to calculate a plurality of level differences between the specific level of each of the plurality of the incident electrical acoustic signal and a plurality of reflected levels corresponding to a plurality of reflected electrical acoustic signals; and
a notification generator adapted to generate an indicator signal representative of status of a middle ear of the user based on the threshold level difference corresponding to each of the plurality of incident electrical acoustic signal.

10. The hearing device according to any of the preceding claims, wherein the notification generator is adapted to transmit the indicator signal to an indicator means for producing a perceivable effect, the perceivable effect selected from a group consisting of a visual signal at the hearing aid device/ remote device like a smartphone, a vibration of the hearing aid device/ remote device like the smartphone, an audio signal using the receiver of the hearing aid device/ speaker of a remote device like a smartphone, text signal at the remote device like smartphone.

11. The hearing device according to any of the preceding claims, wherein the perceivable effect varies in accordance with the magnitude of level difference above the predetermined threshold level.

12. The hearing device according to any of the preceding claims, further comprising a processor that is adapted to adjust at least one parameter, such as gain, of the hearing device based on the measured level difference if the level difference is equal or more than a predetermined threshold level.

13. The hearing device according to any of the preceding claims, wherein the processor is adapted to adjust the parameter of the hearing device within a permissible range.

14. A method for in-situ monitoring of an ear of a user wearing a hearing device, the method comprising
measuring a level difference between a predetermined level of an incident electrical acoustic signal and a reflected level of a reflected acoustic signal, the incident electrical acoustic signal of the predetermined level and a predetermined frequency being impinged on a tympanic membrane of the user and the reflected electrical acoustic signal being reflected from the tympanic membrane in response to the incident electrical acoustic signal; and
activating an indicator means using an indicator signal representative of status of a middle ear of the user if the level difference is equal or more than a predetermined threshold level that is stored in a memory of the hearing device.

15. The method according to claim 14, wherein
the incident electrical acoustic signal is impinged using a receiver of the hearing aid device for a predefined time duration and the reflected electrical acoustic signal is received at a measurement microphone of the hearing aid device, the measurement microphone being different from a microphone of the hearing aid device adapted to capture speech and/ or ambient sound signals; or
the incident electrical acoustic signal is impinged using the receiver of the hearing device for a predefined time duration and the reflected acoustic signal is received at a microphone of the hearing device, further comprising generating the indicator signal representative of status of the middle ear of the user when the feedback value change is equal or more than the threshold feedback value.
